# EUROPEAN PATENT APPLICATION

(11) **EP 1 457 218 A1**
(43) Date of publication of application: **15.09.2004**
(21) Application number: 02790796.3
(22) Date of filing: 18.12.2002
(51) Int. Cl.: A61M 1/14

(54) **AUTOMATIC APPARATUS FOR BLOOD DIALYSIS AND PRIMING METHOD USING THE APPARATUS**

(30) Priority: 18.12.2001 JP 2001385321; 12.12.2002 JP 2002360714
(71) Applicant: JMS Co., Ltd., Hiroshima-shi, Hiroshima 730-8652 (JP); Kitakyushu Institute of Biophysics Co.,Ltd., Kitakyushu-shi, Fukuoka 807-0831 (JP)
(72) Inventor: KIM, Sung-Teh, Yahatanisi-ku, Kitakyushu-shi, Fukuoka 8 (JP); TAOKA, Masahiro c/o Kitakyushu Nefurokuriniku, Kitakyushu-shi, Fukuoka 807-0831 (JP); YAMAMOTO, Chieko, Yahatanisi-ku, Kitakyushu-shi, Fukuoka 8 (JP); YAMANAKA, Kunihiko, Kokurakita-ku, Kitakyushu-shi, Fukuoka 8 (JP); MASAOKA, Katunori c/o JMS CO., ChiyodaFactory, Hiroshima, Hiroshima 731-1514 (JP)
(74) Representative: Gassner, Wolfgang, Dr.
(86) International application number: PCT/JP2002/013216
(87) International publication number: WO 2003/051427

(57) **Abstract**

An apparatus for blood dialysis composed at least of a hemodialyzer (a) whereby blood is brought into contact with a dialysate via a semipermeable membrane and thus purified; a blood circulation system (b) composed of a blood circuit in the arterial side whereby blood is taken out from a patient and flown into the hemodialyzer and another blood circuit in the venous side whereby the blood flown out from the hemodialyzer is returned to the patient; a dialysate supplying/discharging system (c) for supplying and discharging the dialysate composed of a dialysate-supply line for supplying the dialysate to the hemodialyzer and a dialysate-discharge line for discharging the dialysate flown out from the hemodialyzer, and a means (d) of controlling the back-filtration speed. Owing to this constitution, individual steps from the preparation of the dialysis (treatment) to the completion of the treatment and the change from a specific step to the next step can be safely, surely and quickly carried out in most cases. Moreover, a series of therapeutic procedures (i.e., blood collection, initiation of blood dialysis, collection and completion) can be automated.

## Description

### TECHNICAL FIELD

The present invention relates to a hemodialyzing apparatus whereby a series of operations including hemodialysis and corresponding preparation/recovery operations which were used to be performed manually are automated to a maximal extent for labor savings.

### BACKGROUND ART

A hemodialyzer is medical equipment for purifying the blood of renal failure patients and drug intoxication patients. The mechanism for hemodialysis treatment generally consists of three parts: a hemodialyzer, a blood line through which blood circulates, and a dialysate supply system. While maintaining an extracorporeal circulation by means of a blood line which is directly connected to the inside of the blood vessel at two locations (one paracentesis needle inthecase of "single-needle" type), the blood is fed into the compartment inside a hollow fiber of the hemodialyzer, which is connected to some midpoint in the blood line.

On the other hand, an electrolyte solution called dialysate is flown into the compartment outside the hollow fiber of the hemodialyzer in the opposite direction to the blood flow. Both compartments of the hemodialyzer are separated by a separation membrane called hemodialysis membrane, and while the blood and dialysate flow in opposite directions, material transfer by diffusion occurs depending on the density gradient across the separation membrane, and thus removal of uremic toxin and intoxicating substances as well as replenishing of substances in shortage occur. Generally, the above described hemodialyzing apparatus is composedof equipment for controlling the maintenance of extracorporeal circulation, stable supply of dialysate, removal of excess water from blood, and the like.

In view of the monitoring of equipment and patient information during hemodialysis and security management, prior art hemodialys is monitoring apparatuses are excellent; however, they are insufficient in view of labor saving in the overall routines relating to hemodialysis such as the priming before treatment (preparation process for washing and thereby cleaning the blood line and flow path of hemodialyzer), blood withdrawal after needling (operation for starting extracorporeal circulation by withdrawing the blood from the body to the blood line), liquid replenishing during hemodialysis treatment, blood returning in the end (operation of finishing extracorporeal circulation for returning the blood in the blood line into the body), smooth transitions between individual processes, and the like.

Automation has not been achieved especially in specific processes and transitions between processes, and thus labor intensive operations and expertise of medical professionals were needed. To finish the priming, blood withdrawal and blood returning in a short time for a number of patients visiting at the same time, a large amount of manpower needed to be thrown in at a time. On the other hand, such placement of manpower was excessively large during hemodialysis treatment (blood circulation), thereby causing temporal non-uniformity in the content of labor, and economic inefficiency.

Moreover, in conventional hemodialysis practices, about 1 liter of saline, which is an intravenous formulation, was used for cleaning/filling the blood line and the hemodialyzer in the priming process. It has been pointed out that cleaning with the amount of 1 liter is not enough to sufficiently clean the flow path. However, use of a large amount of saline for cleaning/filling will cause an increase in costs.

Moreover, when a drop of blood pressure occurs during hemodialysis treatment, additional saline is needed thus complicating the routine and causing a cost increase. Recently, purifying technology of dialysate has made a remarkable progress, and a new system has been established in which an ultra-pure purified dialysate is applied for back-filtration liquid replenishing. In such a system, purified dialysate can be used in place of saline as a rinse liquid or a replenishing liquid by subjecting it to back-filtration. However, there has not been disclosed a liquid replenishing line system, which is capable of readily and reliably providing back-filtered dialysate for the purpose of liquid replenishing during priming and treatment without resulting in a secondary contamination caused by stagnation of dialysate.

### DISCLOSURE OF THE INVENTION

The present invention is directed to rationalization (automation, simplification, labor saving, time saving, cost cutting) of hemodialysis treatment routines, which were labor intensive, require professional skills, and were considered to be difficult to rationalize, and also to enhancement of the safety of the treatment.

Thus, it is the object of the present invention to provide a hemodialyzing apparatus which allows the automation of each individual process and also most of the transitions from a particular process to next one from hemodialysis preparation to treatment completion in the above described hemodialysis medicine, unlike a conventional apparatus in which only part of each process is automated. Thus, the object is to automatically perform a series of processes from hemodialysis preparation to treatment completion safely, reliably and speedily, and to significantly reduce the labor and supply costs.

The above described technical problems has been successfully solved by the present invention, which is an automatic hemodialyzing apparatus composed of at least: (a) a hemodialyzer for purifying blood by bringing blood and dialysate into contact via a semi-permeable membrane; (b) a blood circulation system for circulating blood, the blood circulation system consisting of an arterial line for deriving blood from the patient to feed it into the hemodialyzer, and a venous line for returning blood, which has flown out from the hemodialyzer, to the patient; (c) a dialysate supply/discharge system for supplying/discharging dialysate, the dialysate supply/discharge system having a dialysate supply line for supplying dialysate to the hemodialyzer, and a dialysate discharge line for discharging the dialysate which has flown out of the hemodialyzer, and (d) back-filtration speed regulation means placed on the dialysate supply/dischage system (c), wherein said automatic hemodialyzing apparatus comprises: a blood pump in at least one of the two blood lines of said blood circulation system, the blood pump being reversibly-rotatable and adjustably cooperate with said back-filtration speed regulation means; a venous chamber in the venous line; an overflow line connected to said venous chamber and having open/close means for making liquid overflow from said blood chamber to outside the blood line; and line open/close means in the downstream and/or the upstream of the linking part of said venous chamber, and also said automatic hemodialyzing apparatus comprises at least one function selected from the group consisting of the following functions:
(1) Function of detecting blood line obstruction during priming (A),
(2) Function of back-filtration for canceling negative pressure in the line during priming (B),
(3) Function of operating line open/close means in the downstream of the venous chamber upon starting blood withdrawal (C),
(4) Function of controlling back-filtration of the dialysate in at least one of priming process, liquid replenishing process and blood returning process (D),
(5) Function of blood-withdrawal failure detection (E),
(6) Function of detecting obstruction of the vein pressure monitor line during hemodialysis (F),
(7) Function of rapid liquid-replenishing in both the arterial and venous directions, or in the vein direction (G),
(8) Function of detecting abnormal internal pressure in the line during blood returning (H),
(9) Function of automatic regulation of the liquid level in the venous chamber (I),
(10) Function of hemodialyzer exchange (J), and
(11) Function of liquid discharge operation during blood line recovery/disposal (K).

Especially, for automating the hemodialyzing apparatus, it is preferable to have, out of the above described functions, the function of controlling back-filtration of the dialysate in at least one of priming process, liquid replenishing process and blood returning process (D), the function of blood-withdrawal failure detection (E), and the function of detecting abnormal internal pressure in the line during blood returning (H).

Furthermore, according to the hemodialyzing apparatus of the present invention, by allowing the cooperative operation of the back-filtration speed or water removing speed of the back-filtration speed regulation means (d) and the liquid feed speed of the blood pump, or the gang control of the above described blood pump, the back-filtration speed regulation means, line open/close means provided in the overflow line and the line open/close means provided in the upstream side and/or the downstream side of the venous chamber connection part, especially the line open/close means provided in the downstream line, it is made possible to perform the priming process before starting hemodialyzing, the blood withdrawal process from the patient to the blood circulation system upon starting hemodialysis, the initiation mechanism, which consists of, as desired, automatic start operation mechanism, starting switch, and the like, for shifting from the blood withdrawal process to the hemodialysis process, the hemodialysis process, and the blood returning process for returning the blood from the blood circulation system to the patient upon completion of hemodialysis, or to perform automatically and continuously each process, thereby further achieving automation of the hemodialyzing apparatus of the present invention.

For example, it is possible to provide a substantially fully automatic hemodialyzing apparatus which can automatically perform the priming process before starting hemodialysis and, after connecting the arterial blood line and the venous blood line of the hemodialyzing apparatus to the paracentesis needle or the like, automatically and continuously perform each process or mechanism of the blood withdrawal process from the patient to the blood circulation system upon starting hemodialysis, the initiation mechanism for shifting from the blood withdrawal process to the hemodialysis process, the hemodialysis process, and the blood returning process for returning the blood from the blood circulation system to the patient upon completion of hemodialysis.

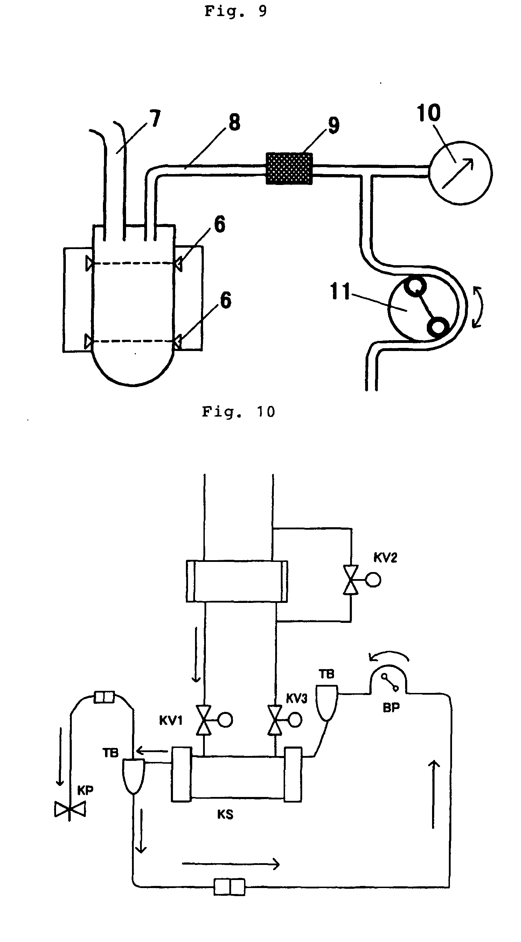

The back-filtration speed regulation means which can be adopted in the hemodialyzing apparatus of the present invention may utilize any conventional means known in the field of the hemodialyzing apparatus without placing any specific limitations and also there is no limitation on its structure or kind as long as it has such functions as to provide the liquid such as dialysate or saline to the hemodialyzer and allow the dialysate distribution system, which discharges the dialysate flow out of the hemodialyzer, to force the foregoing liquid into the hemodialyzer, or to withdraw water or blood from the hemodialyzer, by changing alternately from positive to negative, or negative to positive the difference between the inflow amount into the foregoing hemodialyzer and the outflow amount from the hemodialyzer per unit of time and further allows to arbitrarily regulate the forcing amount of the foregoing liquid or the withdrawal amount of water or blood. Such back-filtration speed regulation means and/or water removal speed regulation means include, for example, methods as described below.
(1) Means described in a previous application of the present applicant in which the dialysate supply line is provided with a side line having a pump (JP, A, 06-114102),
( 2) Means in which supply means and feed means of dialysate of which discharge amount is controllable are provided in the supply flow path of dialysate to the hemodialyzer and the feed flow path of dialysate therefrom, and means for repeatedly increasing the capacity of the dialysate flowpath from the supply means to the feed means via the hemodialyzer is provided in the foregoing dialysate flow path (JP, B, 58-14223),
(3) Means in which a rotary pump capable of regulating the supply amount of dialysate is provided in the supply flow path of dialysate (JP, A, 61-13968),
(4) It is possible to provide three compartments as a supply/discharge system of dialysate in a diaphragm chamber for supplying and discharging dialysate and drive a water removing (or supply) pump in communication with a central chamber to such or discharge liquid into or from the central chamber, thereby allowing to perform back-filtration or water removing via the dialysate supply/discharge system and a hemodialyzer (JP, A, 2001-37873, JP, A, 2001-37872).
(5) It is also possible to provide an open/close valve as shown in Fig. 10 in the supply line or the discharge line of dialysate, and perform back-filtration or water removing by gang control of the foregoing open/close valve (and the supply/discharge chamber for substantially equalizing the liquid supply and liquid discharge linked to the supply/discharge lines).

In the above described mechanism (1), a single liquid feed means may be used both for water removing and liquid replenishing for the blood circulation system by providing a third liquid-feed, back-filtration speed regulating means which can supply liquid both forwardly-and-backwardly in at least one of the first bypass line which bypasses the upstream side and the downstream side of the first liquid feed means provided in the dialysate supply line of the dialysate supply/discharge system and/or the second bypass line which bypasses the upstream side and the downstream side of the second liquid feed means provided in the dialysate discharge line.

That is, placing a water flow pump in the first bypass line provided in the dialysate supply side and feeding liquid in the same direction as the first liquid feed means (dialysate supply pump) will cause the amount of dialysate flowing into the hemodialyzer to become more than the amount of liquid flowing out from the hemodialyzer, resulting in liquid replenishing to the blood circulation system. When liquid is fed in the direction opposite to the foregoing, the liquid inflow amount into the hemodialyzer becomes smaller than the liquid outflow amount, resulting in water removing from the blood circulation system.

The above described mechanism can be achieved similarly by placing a water flow pump in the second bypass line provided in the dialysate discharge side and switching the liquid feed direction. In this case, when liquid feed is performed in the same direction as the second liquid feed means (dialysate discharge pump), the discharge amount flowing out from the hemodialyzer becomes larger than the liquid amount flowing into the hemodialyzer, such that water removing from the blood circulation system is performed. When liquid feed is reversed, in the contrary, the outflow amount from the hemodialyzer becomes smaller than the inflow amount such that liquid replenishing to the blood circulation system occurs. The embodiment in which a pump is provided in the second bypass line to perform liquid replenishing or water removing is preferable for a personal hemodialyzing apparatus of which dialysate flow amount is limited. When applying for a personal hemodialyzing apparatus, although the pump may be placed only in the second bypass line, it is possible to increase the liquid feeding capacity of liquid replenishing or water removing by placing another pump in the first bypass line.

In the above described embodiment for performing liquid replenishing or water removing, particularly complex structures for water removing/liquid replenishing are not required. The dialysate feed line and the bypass line are simple, and providing the above described third liquid feed means in either one of the two bypass lines has the advantage of making the stagnation of dialysate less likely to occur. That is, since the third liquid feed means provided in the dialysate feed system almost incessantly operates in all the processes of hemodialysis, substantially no stagnation will occur in the foregoing bypass line. Constant liquid flow maintained in the line suppresses the secondary bacterial growth of the dialysate thereby avoiding endotoxin contamination. Moreover, providing the foregoing third liquid feed means in the first bypass line prevents the dialysate discharge from flowing into the bypass line thereby eliminating the risks of pollution of bypass lines and clogging of the line due to the wastes in the discharge liquid.

The liquid feed capacity of the back-filtration speed regulation means in the automatic hemodialyzing apparatus of the present invention is to be specified relating to the liquid feed amount of the blood pump provided in the blood circulation system, and a liquid feed amount (liquid feed capacity) of 0 to 600 ml/min is preferable. Part (about half) of the dialysate which is back-filtered by a hollow fiber and flows into the blood line is preferably made to flow into one blood line by a blood pump, and the remaining dialysate (half) is made to flow into the other blood line, and for that purpose, the liquid feed amount of the blood pump is preferably adjusted to be within a range of 0.1 to 1.5, preferably 0.4 to 1.1 with respect to the liquid feed amount of the back-filtration speed regulation means.

In the hemodialyzing apparatus of the present invention, it is preferable that a venous pressure monitor line is provided in the venous chamber, and an air-bubble sensor is provided in the blood line. The above described venous pressure monitor line is configured, for example, such that an open/close means which can close and open a communicating conduit through the connecting conduit, and an air reservoir chamber in communication with the foregoing open/close means are provided in the upper part of the venous chamber C as shown in Fig. 8, and a venous pressure monitor line is provided in the foregoing communicating conduit which is branched off between the foregoing closing means and the venous chamber.

The hemodialyzing apparatus of the present invention enables the blood withdrawal from the artery side (blood withdrawal process A) by overflow automatic blood withdrawal from the overflow line by providing line open/close means in the downstream line of the venous chamber connection part, and controlling this open/close means and blood pump, back-filtration speed regulation means, line open/close means provided in the overflow line, and line open/close means provided in the downstream line of the venous chamber connection part.

Since when performing blood withdrawal process by withdrawing blood from both of the vein side and artery side, or withdrawing from vein side (blood withdrawal process B), if an anomaly occurs in the blood withdrawal of vein side, it is possible to automatically and immediately shift to blood withdrawal only through blood withdrawal process A by the gang control of the blood pump, back-filtration speed regulationmeans, line open/close means provided in the overflow line, and line open/close means provided in the downstream line of the venous chamber connection part, this overflow automatic blood withdrawal function is a very effective function for automating the hemodialyzing apparatus. Moreover, although blood withdrawal from vein side is difficult for some patients, this overflow automatic blood withdrawal function can be applied to blood withdrawal from such patients and, in addition to that, since blood withdrawal is performed only by the blood pump with the water removing/liquid replenishing pump being stopped, the function is also advantageous in that the hemodialysis membrane will not be subject to an excess negative pressure. Shifting to blood withdrawal process only with this blood withdrawal process A may be performed, for example, by automatically equalizing the speed of the pump with the water removing speed.

However, the automatic hemodialyzing apparatus of the present invention does no need to be provided with an overflow line when the overflow automatic blood withdrawal function as described above is not necessary, and even if the overflow line is always kept closed, automation to some extent can be achieved.

The hemodialyzing apparatus which adopted the above described configuration of the present invention was able to achieve the purpose of safely, securely, and rapidly performing each process from hemodialysis preparation to treatment completion, and also most of the transitions from a particular process to a next process of these processes, thereby significantly reducing the costs of labor and consumable items.

Hereinafter, the above described functions (A) to (K) will be concretely described.

### Function of detecting blood line obstruction during automatic priming (A)

In automatic priming, if obstruction of a blood line occurs, normal priming becomes unable to be performed. As a monitoring function to cope with this, it is preferable to install at least one of following functions of detecting blood line obstruction during automatic priming.
(1) A function of enabling an air-bubble sensor for a fixed period of time, preferably for 1 to 3 seconds, in an automatic priming process to check if the blood line is filled with the priming liquid and determining that a blood line obstruction has occurred upon detecting air (generation of air buffle warning).
(2) A function of detecting an obstruction of the blood line by a liquid pressure warning during automatic priming, in which obstruction of the blood line will result in warning display and alarm generation. However, since there is trade off with liquid pressure control for filtration speed, such liquid pressure state for the first time will not generate a warning.

### Function of back-filtration operation for canceling negative pressure during automatic priming (B)

In priming in a circulation state (closed system) by the dialysate, the liquid temperature is slightly lower in return liquid from the hemodialyzer than in liquid feed side of hemodialyzer, which results in variation of the density of the dialysate, causing a slight withdrawing (negative pressure) tendency. When such a negative pressure tendency results, especially in a priming process in a recirculation state, air may be mixed into the line if the connection of the blood line is not sufficient, or the venous chamber may be collapsed. To prevent such a problem, the functionof back-filtrationoperation for canceling negative pressure during automatic priming (B) allows to maintain the inside of the blood line to be positive pressure by performing a small amount of back-filtration even in a blood line circulating state of the priming liquid in priming, for example, performing back-filtration by 10 ml every 10 minutes (back-filtration speed of 100ml/min) or performing similar back-filtration by use of hemodialyzing liquid pressure.

### Function of delaying vein side clamp operation upon starting blood withdrawal (C)

After completing the priming process, if the blood line arterial/venous bypass part for priming is separated before starting automatic blood withdrawal, air-bubbles may be mixed into the front end part of the blood line due to liquid spill, or the like. If blood withdrawal is started in such a situation, since there is risk that air bubbles may be mixed into the patient side especially when the line internal pressure is high on the vein side (positive pressure), it is possible to prevent air-bubbles from mixing into the patient side by starting automatic blood withdrawal using a water removal method with the line open/close means (clamp) in the downstream of the venous chamber being closed before starting blood withdrawal, and opening the foregoing clamp below the venous chamber in a predetermined time period, preferably 1 to 3 seconds after the automatic blood withdrawal to make the blood line internal pressure to be negative.

### Function of controlling back-filtration operation in at least one of priming process, liquid replenishing process and blood returning process (D)

The back-filtration speed during the priming process, liquid replenishing process and blood returning process is significantly affected by the ultrafiltration rate (UFR) of the hemodialyzer. That is, when the setting of the back-filtration speed is higher than an appropriate ultrafiltration rate (UFR) or membrane permeating performance of the hemodialyzer, since the dialysate pressure will increase and thereby a liquid pressure warning will be generated making it impossible to perform smooth back-filtration operation, the back-filtration speed must be lowered when the ultrafiltration rate or the membrane permeability is low, and conversely when the ultrafiltration rate or the membrane permeability is high, the back-filtration speed may be increased.

The control of the above described back-filtration is preferably performed by a method with which a feedback control of the back-filtration speed based on the dialysate pressure may be possible for each of the foregoing back-filtration processes, that is a feedback control (constant pressure control) performed while keeping the liquid pressure for the back-filtration speed constant is preferable. For this reason, in the automatic hemodialyzing apparatus of the present invention, it is preferable to perform back-filtration operation by automatically setting an appropriate back-filtration speed by performing a feedback control of back-filtration speed based on the measurements of dialysate pressure measured by use of a liquid pressure sensor of the hemodialyzer (console). For example, in the case of a hemodialyzer having a low membrane permeability, it is preferable to lower the back-filtration speed and perform back-filtration operation in automatic priming process, liquid replenishing process and blood returning process.

Furthermore, in the automatic liquid replenishing and automatic blood returning processes, unlike during priming, since the effective membrane area of the hemodialyzer decreases due to wastes, fouling, blood coagulation, etc. as the hemodialysis process proceeds, and thereby the back-filtration speed specified for the liquid pressure feedback control may be decreased lower than the settable back-filtration speed during priming, it is preferable to store a back-filtration speed, which is set after the priming process is stabilized, as a maximum value and to use it as the maximum value of the back-filtration speed for the feedback control in the automatic liquid replenishing and automatic blood returning processes described later.

Moreover, in the case of a dry-type hemodialyzer, since the membrane surface is coated with glycerin or the like, or from some other reasons, the back-filtration speed in the early stage of the priming would decrease lower than a value with a UFR.

Further, other than feedback control (constant pressure) methods, a control method (speed control) may be used with which the set value for liquid pressure control in automatic liquid replenishing and automatic blood returning processes is specified in advance, and the set back-filtration speed is decreased by a fixed rate or by a fixed speed as a liquid pressure measurement reaches this set value.

In the control of automatic priming, automatic liquid replenishing, and automatic blood returning processes by the above described back-filtration, in addition to the constant pressure control system or the speed control system, a control method of switching from the constant pressure control system to the speed control system may be adopted, and further control may be performed by appropriately selecting the aforementioned control systems, and switching these selected control systems.

### The function of blood-withdrawal failure detection (E)

The function of blood-withdrawal failure detection (E) includes an artery side blood-withdrawal failure detection function, a vein side blood-withdrawal failure detection function, and a combination of both functions as described below, and typically the combination of aforementioned both functions is utilized.

Since there is a blood pump installed in the artery line, it is impossible to detect genuine arterial pressure. Conventionally, a blood-withdrawal failure state in the artery side was determined by visually inspecting a pillow of blood line or detecting the collapse of the pillow with a pillow sensor, or determined based on the convulsion of blood pump segment. On the other hand, the automatic hemodialyzing apparatus of the present invention is provided with an artery side blood-withdrawal failure detection function to be described below, and this function makes it possible not only to detect an artery side blood-withdrawal failure state, but also control such a state representing it with numerical data, while conventional detection methods such as visual inspection and a pillow sensor can not represent such states numerically. The function may also achieve such effects as preventing the formation of a thrombus by eliminating the pillow and thereby reducing the steps inside the blood line.

In a blood withdrawing state or a hemodialyzing state, with the venous line being closed for a set period of time, for example about 1 to 10 seconds, preferably 3 to 5 seconds by means of lose means (for example, a clamp) provided in the downstream side of the venous chamber in the venous line, and with the blood pump being operated in this closed state and water removing pump being stopped (the water removing pump being stopped only in the case of blood withdrawal with water removing), variations in the vein pressure are detected to detect an artery side blood-withdrawal failure based on the foregoing detection result. When blood withdrawal is normally performed, the vein pressure is to increase.

The hemodialyzing apparatus of the present invention preferably has a following vein side blood-withdrawal failure detection function in addition to the abovementioned artery side blood-withdrawal failure detection function in the automatic blood withdrawal process.

Providing a vein side blood-withdrawal failure detection function (Z-1) or (Z-2) as shown belowwill enable early detection of anomalies such as blood-withdrawal failures, blood coagulation, and narrowing or obstruction of the blood line.

In a blood withdrawal process by blood withdrawal with water removing, in a state in which blood withdrawal from a vein such as a cutaneous vein can not be performed, it is determined that a blood-withdrawal failure state has occurred when the dialysate pressure becomes an excessively negative pressure state after starting the blood withdrawal operation (an excessively negative pressure state is measured by providing a pressure sensor in the dialysate discharge side, and the pressure reference corresponding to the excessively negative pressure can be altered by internal setting) (Z-1).

In vein side blood withdrawal, blood pump is temporarily stopped to decrease the water removing speed by the equivalent amount corresponding to the blood pump speed and the vein side blood withdrawal is continued. In this state, when the vein pressure becomes lower than a lower limit, or the liquid pressure becomes lower than a lower limit, it is determined that a vein side blood-withdrawal failure has occurred (Z-2).

In above described automatic blood withdrawal, it is possible to measure the excessively negative pressure state of the dialysate pressure, and the excessively negative pressure state of the vein pressure, concurrently with specific negative pressure values, and upon detecting an aforementioned vein side blood-withdrawal failure state, blood withdrawal with water removing is continued only by blood withdrawal from the artery side with the blood pump speed being the same as the water removing speed or faster than the same speed by less than about 10% .

### Function of detecting obstruction of vein pressure monitor line (F)

After starting hemodialyzing, if aforementioned vein pressure monitor line is kept closed, the vein pressure cannot be measured and therefore warning function does not act. Therefore, it is also preferable to add a function of detecting obstruction for vein pressure monitor line (F) to the hemodialyzing apparatus of the present invention.

The function of detecting obstruction of vein pressure monitor line (F) may be configured such that if the measurement of vein pressure does not vary within a predetermined range in a predetermined period of time, for example within 30 to 60 sec after starting hemodialyzing even though the vein pressure measurements should vary since there is a pulsation due to the blood pump, a warning may be generated indicating that obstruction in the vein monitor line has occurred.

### Function of rapid liquid-replenishing in both arterial and venous directions, or in the vein direction alone (G)

The hemodialyzing apparatus of the present invention preferably has a function of rapid liquid-replenishing in both arterial and venous directions, or in the vein direction alone (G). This rapid liquid replenishing function (G) is emergency treatment means to cope with a blood pressure drop of the patient, or the like. In this case, since it is the purpose to increase the amount of blood circulation in the patient, it is made possible to increase the amount of blood circulation in the patient not only by injecting replenishing liquid into the vein side, but also by injecting from the artery side by reversely rotating the blood pump. Since, especially in an inner shunt, both the artery and the vein are one and the same blood vessel, the amount of blood circulation can be readily increased by supplying liquid from both the artery and the vein. Moreover, rapid liquid-replenishing function during the forward rotation and stoppage of the blood pump can be carried out by injection only from the vein side by supplying the hemodialyzer with dialysate by means of back-filtration speed regulating means provided in the dialysate supply/discharge system.

### Function of detecting abnormal internal pressure in the line during blood returning (H)

The automatic hemodialyzing apparatus of the present invention preferably has a function of detecting abnormal internal pressure in the line during blood returning (H). This function of detecting abnormal internal pressure in the line during blood returning (H) is capable of measuring the dialysate pres sure in the dialysate line and the vein pressure in the venous line with pressuremeasuringmeans after starting blood returning in the blood returning process to determine that an obstruction of the venous line has occurred when the vein pressure in the venous line goes into an increasing trend from a stable state, thereby identifying line internal pressure anomalies. In contrast, since no artery pressure monitor is provided for detecting an obstruction in the artery side from the abovementioned reason, an obstruction of the artery side is detected from a rising trend from a stable sate of each pressure of the dialysate pressure and the vein pressure by starting the monitoring of the dialysate pressure and the vein pressure after having started blood returning. Also, in the above described function of detecting abnormal internal pressure in the line during blood returning, although an obstruction may occur without showing a stable state in early stages of blood returning, in such a case, an anomaly state is determined from the vein pressure, or a change amount Δ or an absolute pressure of the dialysate pressure and the vein pressure.

### Function of automatic regulation of the liquid level in the venous chamber (I)

Operations such as liquid level regulation of the blood chamber upon starting hemodialysis, monitoring and liquid level regulation of the chamber during hemodialysis, and lowering the liquid level of the blood chamber during blood recovering (or blood returning) after completion of hemodialysis used to be performed manually by medical staff. However, since such manual operation of monitoring and liquid level regulation of the blood chamber not only takes much time but also involves difficulty in accurately regulating the liquid level, there is risk that air is mixed into the patient side when, for example, the liquid level of the blood chamber during hemodialysis is lowered below a predetermined level.

In contrast to this, since the automatic hemodialyzing apparatus of the present invention makes it possible to automatically perform the operation of monitoring and liquid level regulation of the blood chamber as described below unlike conventional manual operation of monitoring and liquid level regulation of the blood chamber, it is effective in achieving full automation of the automatic hemodialyzing apparatus of the present invention. Further, the monitoring and liquid level regulation of the blood chamber as described below are not only effective in full automation of the automatic hemodialyzing apparatus, but also capable of lowering the liquid level of the blood chamber during blood returning (or blood recovering) after completion of hemodialysis thereby reducing the use amount of saline or dialysate.

As the above-described monitoring and liquid level regulation means of the blood chamber, there is provided with, for example, a air reservoir chamber 3 having lose means (clamp) 4 in communication with the connecting conduit which constitutes a vein monitor line S4 above the vein chamber as shown in Fig. 8. Moreover, the vein pressure monitor 5 is in communication with the above-described connecting conduit, which is in communication with the above-described lose means (clamp) and the venous chamber, via a branch part.

Further, another embodiment of the above-described monitoring and liquid level regulationmeans of the blood chamber may be configured, as shown in Fig. 9, to have a liquid level detection sensor provided in the venous chamber, a conduit which is in communication with the venous chamber on one end and with the ambient air on the other end, and a tubing pump rotatable in both directions which is provided in said conduit, and to be able to regulate the liquid level in the venous chamber by switching the rotation of the tubing pump in either direction based on the detection result of the above-described liquid level detection sensor.

In the blood returning process, to minimize the amount of dialysate required by back-filtration, it is necessary to reduce the amount of blood, that is, liquid level remained in the vein chamber during blood returning as low as possible within a safe range. Since the hemodialyzing apparatus of the present invention can automatically perform the regulation of the amount of blood, i.e., the liquid level remained in the vein chamber without resorting to manual operation, it is not only able to lower the liquid level of the venous chamber during blood recovering (or blood returning) after completion of hemodialyzing thereby reducing the use amount of saline or dialysate, but also effective in achieving the automation of the hemodialyzing apparatus.

Providing liquid level detection means, for example, a liquid level detection sensor in the venous chamber especially as shown in Fig. 9 and thereby automatically performing the monitoring and the regulation of the liquid level of the blood chamber will be further effective in fully automating the automatic hemodialyzing apparatus.

### Function of hemodialyzer exchange (J)

The automatic hemodialyzing apparatus of the present invention preferably has a function of hemodialyzer exchange.

Conventionally, when a leakage of a hemodialyzerwas found, the hemodialyzer was exchanged by temporarily performing blood returning operation. In contrast, in this function, when a leakage of the hemodialyzer is found, the hemodialyzer is exchanged while temporally stopping the operation of the blood pump and keeping the blood in the hemodialyzer to be pushed out in both artery and vein directions by a blood returning operation through back-filtration. After exchanging the hemodialyzer by exploiting this function, the automatic hemodialyzing apparatus of the present invention removes pure water, glycerin, air bubbles, and the like by high-performance water removing operation, and then moves to the hemodialysis process. This function of hemodialyzer exchange (J) is also extremely effective means for automating the automatic hemodialyzing apparatus of the present invention.

### Function of liquid discharge operation during blood line recovery/disposal (K)

In an existing method, when detaching and discharging the blood line/hemodialyzer from the hemodialyzing unit (console), saline (dialysate) within the blood line is discarded as it is. The cost for disposal is generally determined depending on the volume; however, in some regions, it is determined based on the weight. Even when the disposal cost is based on volume, withdrawing the saline thereby reducing the weight will make it possible to improve both workability and safety in, for example, handling of the disposal case and the like in medical facilities.

In the hemodialyzing apparatus of the present invention, the function of liquid discharge operation during blood line recovery/disposal (K) makes it possible to draw out saline in the line after completing blood returning by, connecting the artery and vein tips of the blood line for bypassing, detaching the overflow line or opening the line open/close means provided in the overflow line, and performing water removing operation. Thereafter, by drawing out the dialysate of the hemodialyzer into the console side utilizing the fall, it is made possible to reduce the weight of the waste.

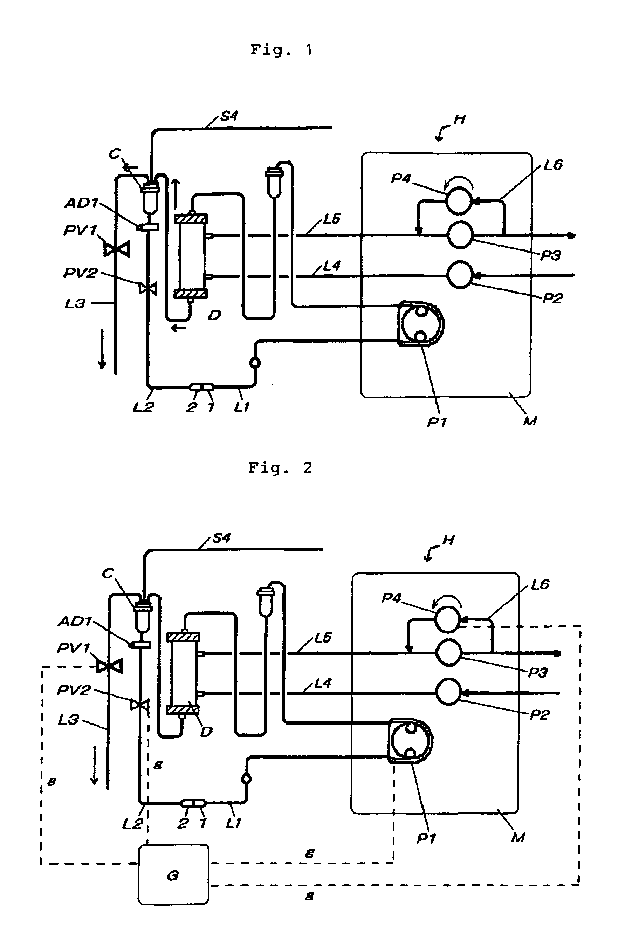

In the present invention, an ultrapure dialysate is preferably used as the dialysate, and the hemodialyzing apparatus of the present invention is preferably fed with the ultrapure dialysate in a stable fashion during hemodialysis operation. For example, it is possible to supply dialysate in a stable fashion by filtering a dialysate supplied from a normal dialysate producing apparatus with an ultrafiltration filter provided in the entrance part, and the like (may be provided in other parts) of the hemodialyzing apparatus (H) relating to the present invention as in Fig. 1 to remove impurities such as dissolved endotoxin and bacteria. Also, dialysate supplied from a typical dialysate producing apparatus is preferably purified in advance conforming to a predetermined water quality standard.

Hereinafter, the configuration of the automatic hemodialyzing apparatus will be described referring to drawings.

### Hemodialyzing console (M)

The hemodialyzing console (M) shown in each figure is a dialysate flow rate regulator, and also a blood flow rate regulator. The hemodialyzing console (M) has typical performances as a dialysate supply mechanism having a closed system.

The console (M) is provided with third liquid-feed back-filtration speed regulation means (hereinafter referred to as water removing/liquid replenishing pump) (P4) to realize a function of water removing/liquid replenishing from/to the blood circulation system by a back-filtration of the hemodialyzer. That is, between the upstream side and the downstream side of the dialysate discharge pump (P3), which is the second liquid feed means for the dialysate discharge line L5, a second bypass line L6 is provided linking both sides, and a water removing/liquid replenishing pump (P4) is installed in this bypass L6. This water removing/liquid replenishing pump (P4) is a fluid pump capable of switching the ejection direction into forward and backward directions and of regulating the flow rate within a range of, for example, about 0 to 500 ml/min, preferably about 0 to 400 ml/min.

Further, although, in each figure, the above described water removing/liquid replenishing pump (P4) is provided in the second bypass line L6 on the dialysate discharge line L5 side, the above described water removing/liquid replenishing pump (P4) may be provided by forming a bypass line between the upstream side and the downstream side of the dialysate discharge pump (P2) linking both sides on the dialysate discharge line L4 and providing the water removing/liquid replenishing pump on this bypass line, or further, the water removing/liquid replenishing pump may be provided in both of the above described bypass lines. Blood line [arterial blood line (L1) and venous blood line (L2)]

The blood line consists of two parts: the arterial blood line (L1) and the venous blood line (L2) as shown in each figure.

The arterial blood line (L1) has a connection part (1) with the arterial side paracentesis needle and a connecting part with the hemodialyzer D. The blood line is provided with a blood pump (P1) which is capable of controlling forward and backward revolution to maintain an extracorporeal circulation.

The venous line (L2) consists of a connecting part with the hemodialyzer D, a venous chamber (C), an overflow line (L3) and vein pressure (blood pressure) monitoring line (S4) which are in communication with the upper part of the venous chamber, and a connecting part (2) with the venous side paracentesis needle.

Also the blood line preferably has an air-bubble detector (AD1) and an air-bubble detector (AD2). The air-bubble detector (AD1) is provided, for example, in the downstream side of the venous chamber so that when air is sensed while returning blood during hemodialysis, the blood pump (P1) is immediately stopped and the valve (PV2) is closed to prevent air frombeing erroneously injected into the body.

### Two valves (PV1 and PV2)

The hemodialyzing apparatus preferably have two valves (PV1 and PV2) for each of automatic priming process, automatic hemodialysis process, automatic blood returning process, or making these processes continuous. The valve 2 (PV2) is provided in the venous line on the downstream side of the venous chamber, and the valve 1 (PV1) is provided in the overflow line (L3).

### Blood chamber

The hemodialyzing apparatus is provided with at least a venous chamber as the blood chamber; however, an arterial chamber in addition to the venous chamber may be provided.

The venous chamber may be provided with, as described above, an overflow line (L3), a vein pressure monitor line and an automatic liquid-level control means for the blood chamber.

The above described automatic liquid-level control means of the blood chamber includes, for example, a method using an air reservoir chamber and means using a tubing pump.

In the automatic liquid-level control means for the blood chamber us ing an air reservoir chamber, the air reservoir chamber 3 is filled with air during automatic priming, for example, as shown in Fig. 8, by opening the open/close means of the connecting conduit. During blood withdrawal process and hemodialysis, the lose means is closed to keep the above described air reservoir chamber 3 filled with air. Then, when starting blood returning, the lose means is opened and the air in the above described air reservoir chamber 3 is fed into the venous chamber C thereby lowering the liquid level of the venous chamber C.

The automatic liquid-level control means of the blood chamber further includes means using a tubing pump as shown in Fig. 9.

The regulation means of the liquid-level in the venous chamber is configured to have a liquid-level detection sensor provided in the venous chamber, a conduit one end of which is in communication with the venous chamber and the other end is in communication with ambient air, and a tubing pump rotatable in both directions which is provided in said conduit, and is characterized in that the rotation of said tubing pump is switched to either side to regulate the liquid level in the venous chamber based on the detection result of the above described liquid-level detection sensor.

For the automation of the hemodialyzing apparatus of the present invention, it is essential to gang control, the blood pump, thewater removing/liquid replenishing pump, and the valve which is the open/close means for the venous blood line and the valve which is the open/close means for the overflow line. Therefore, it is preferable to provide control means which is able to operate these components in conjunction with each other and to control each component in accordance with changes of other components, and transfer means for communicating with each component. The control means is preferably provided in a hemodialyzing console which contains various monitoring devices and safety devices for a general hemodialyzing apparatus.

### BRIEF DESCRIPTION OF THE DRAWINGS

Fig.1 and Fig. 2 are schematic diagrams to show a typical configuration of the automatic hemodialyzing apparatus of the present invention.
Fig. 3 is a schematic diagram to show a flow path of the automatic priming process which utilizes the automatic hemodialyzing apparatus of Fig. 1.
Fig. 4 is a schematic diagram to show another flow path of the automatic priming process which utilizes the automatic hemodialyzing apparatus of Fig. 1.
Fig. 5 is a schematic diagram to show a flow path of automatic blood withdrawal (blood withdrawal with high-performance water removing) process using the automatic hemodialyzing apparatus of Fig. 1.
Fig. 6 is a schematic diagram to show a flow path of the hemodialysis process using the automatic hemodialyzing apparatus of Fig. 1.
Fig. 7 is a schematic diagram to show a flow path of the automatic blood withdrawal (over flow discharge blood withdrawal) process using the automatic hemodialyzing apparatus of Fig. 1.
Fig. 8 is a schematic diagram to show a flow path of the automatic blood returning process using the automatic hemodialyzing apparatus of Fig. 1.
Fig. 9 shows one embodiment of the liquid-level automatic regulation means of the venous chamber.
Fig. 10 shows the configuration of an automatic hemodialyzing apparatus having an embodiment of back-filtration speed regulation means which is different from one shown in Figs. 1 to 8, said figure showing a priming/cleaning process.

In each of the above described figures, H denotes Automatic hemodialyzing apparatus, G Control means, g Transfer system, AD1 Air-bubble detector 1, C Venous chamber, D Hemodialyzer, L1 Arterial blood line, L2 Venous blood line, L3 Overflow line, L4 Dialysate supply line, L5 Dialysate discharge line, L6 Water removing/liquid replenishing bypass line (second bypass line), M Dialysate flow rate regulation apparatus, P1 Blood pump, P2 Dialysate supply pump (first liquid feed means), P3 Dialysate discharge pump (second liquid feed means), P4 Water removal/liquid replenishing pump (back-filtration speed regulation means), V1 Valve 1 (open/close means), V2 Valve 2 (open/close means), S4 Vein pressure monitor line, 1 Connecting part of the artery side paracentesis needle and the artery blood line (L1), 2 Connection point between the vein blood line (L2) and the vein side paracentesis needle, 3 Air reservoir chamber, 4 Lose means (clamp), 5 Branch part of the vein pressure monitor, 6 Level sensor, 7 Overflow line, 8 Air intake/discharge pipe, 9 Air cleaning filter, 10 Vein pressure monitor, 11 Tubing pump, respectively. Also, in Fig. 10, KV1 denotes Liquid feed electromagnetic valve (open state), KV2 Atmospheric open electromagnetic valve (open state), KV3 Return port electromagnetic valve, KS Hemodialyzer, KP Clamp, TB Chamber, BP Blood pump, respectively.

### BEST MODES FOR CARRYING OUT THE INVENTION

### (1) Automatic priming process

A loop is formed by connecting the arterial line L1 and the venous line L2 of the blood line. The dialysate lines L4, L5 are connected to the hemodialyzer D. The console M starts operating in a preparation mode (a mode in which air is removed from the apparatus and the lines, and replacement of liquid with dialysate is performed). Upon completion of preparation, the valve 1 (PV1) is opened and the valve 2 (PV2) is closed while keeping the blood pump P1 stopped. The water removing/liquid replenishing pump P4 is operated to perform the back-filtration of the dialysate at 400 ml/min by internal setting to feed dialysate into the blood line via the hemodialyzer D. Through this operation, the flow path between the hemodialyzer D and the venous chamber C is cleaned by discharging the liquid in the foregoing flow path from the overflow line L3 (hereinafter referred to as process A)(see Fig. 1). The pump P2 and pump P3 of the dialysate lines L4, L5 are controlled in such a way that the liquid supply amount and the liquid discharge amount are synchronized.

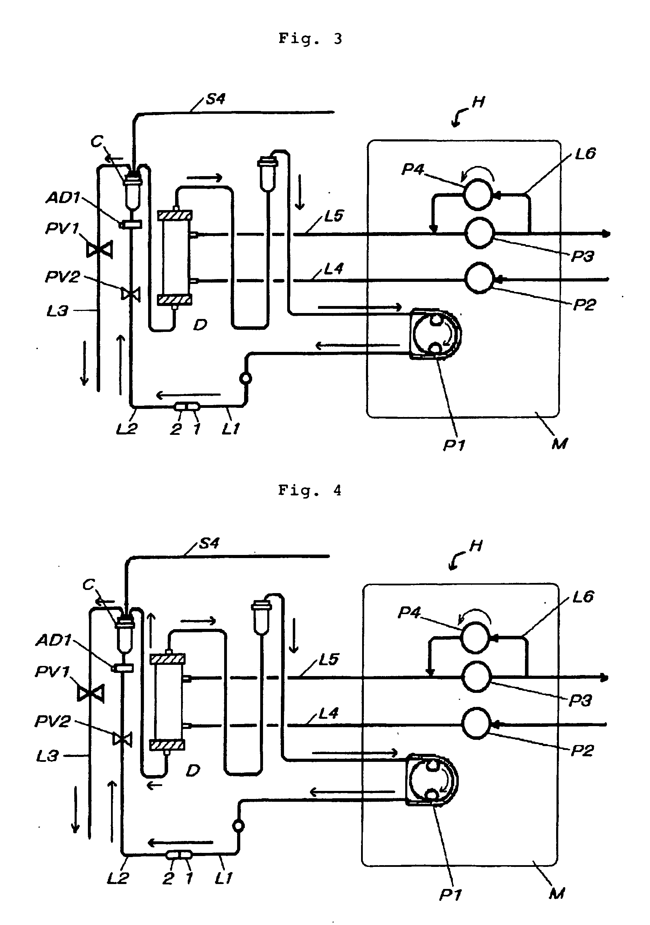

With the valve 1 (PV1) and the valve 2 (VP2) being opened, the water removing/liquid replenishing pump 4 is operated to perform the back-filtration of the dialysate at 400 ml/min by internal setting to feed dialysate into the blood line (liquid replenishing). And the blood pump P1 is backwardly (opposite to the liquid feed direction of the blood pump during hemodialysis) rotated at 400 ml/min (or 90% to 100%) as with the pump P4 thereby cleaning the arterial line L1 and the part of the venous line L2 from the connecting part 2 to the venous chamber C while discharging from the overflow line L3 the dialysate supplied into the blood line by the water removing/liquid replenishing pump 4 (hereinafter referred to as process B)(see Fig. 3).

Further, performing chucking (flushing) operation by open/close the valve V2 (PV2) during liquid injection in the automatic priming process will make it possible to remove the air accumulated in the venous chamber mesh part.

The air-bubble sensor is activated for 1 to 3 seconds, 30 to 60 seconds after the dialysate has circulated throughout the blood line, to check if the inside of the blood line is filled with the priming liquid, and upon detection of air (air-bubble warning generation) it is determined that a blood line obstruction has occurred (function of detecting blood line obstruction during automatic priming (A)).

With the valve 1 (PV1) and valve 2 (PV2) being opened, the blood pump 1 is backwardly rotated setting its liquid feed speed at 50 % of the back-filtration speed, i.e. 200 ml/min (internal setting). Also, with the water removing/liquid replenishing pump P4 internally set at 400 ml/min (internal setting), the dialysate is back-filtered thereby circulating a half amount of the dialysate replenished by the pump into the upstream side of the hemodialyzer, and the other half amount of the dialysate into the downstream side of the hemodialyzer, and thus the entire blood line is cleaned while discharging the liquid from the overflow line L3 (hereinafter referred to as process C)(Fig. 4). Further, performing chucking (flushing) operation by open/close the valve 2 (PV2) during liquid injection in the automatic priming process will make it possible to remove the air accumulated in the venous chamber mesh part.

The automatic priming process adopted in the present invention includes, in addition to one consisting of the above described process A, process B an process C (priming method 1), one consisting of process B, process A and process C (priming method 2), one consisting of process C alone (priming method 3), one consisting of process C and process D (which is a process in which the entire blood line is cleaned while circulating the liquid existing in the foregoing line) (priming method 4), one consisting of the foregoing priming method 1 in combination with the above mentioned process D (priming method 5), or one consisting of the foregoing priming method 2 in combination with the foregoing process D (priming method 6).

Further, in priming in which the dialysate is in a circulating state (closed system), the liquid temperature in the returning fluid from the hemodialyzer is slightly lower than the liquid temperature of the feeding fluid to the hemodialyzer resulting in a variation of the density of the dialysate, and this tends to cause slight drawing effect (negative pressure). When such a negative pressure occurs, especially in a recirculation state of priming process, air will be mixed into the line if the connection of the blood line is insufficient, or the venous chamber will be deformed. In order to prevent the above describe problem, the automatic hemodialyzing apparatus of the present invention makes it possible to maintain positive pressure tendency in the blood line by periodically performing a small amount of back-filtration even in a blood line circulating state of the priming liquid in priming, for example, by performing a back-filtration of 10 ml every 10 minutes (back-filtration speed 100ml/min), or performing similar back-filtration by the dialysate pressure (function of back-filtration opertion for canceling negative pressure in the line during automatic priming (B)).

With the valve 1 (PV1) being closed, the valve 2 (PV2) is opened to stop the water removing/liquid replenishing pump 4. The blood pump P1 is kept rotating in the forward direction at a liquid feed speed of 350 ml/min (internal setting) in a circulating, waiting mode (process D). Alternatively, the blood pump is stopped after a predetermined time has elapsed to complete the priming process. After the completion of the automatic priming, tip parts 1, 2 of the blood lines are detached and each tip part is connected to the paracentesis needle in the vein and artery of the patient.

Further, after completing the priming process, if the artery-vein bypass part of the blood line is separated for priming before starting automatic blood withdrawal, air bubbles may be introduced into the tip part of the blood line due to liquid spill. Because if blood withdrawal is started in this state, especially when the internal line pressure is higher in venous side (positive pressure), there is a risk of air bubble coming into the patient side, mixing of air bubbles into the patient side is prevented by, prior to start blood withdrawal, closing the clamp in the downstream of the venous chamber to start automatic blood withdrawal in a water removing method, and by opening the above mentioned clamp in the downstream of the venous chamber after a predetermined time has elapsed after the automatic blood withdrawal, preferably after 1 to 3 seconds after the automatic blood withdrawal (function of delaying clamp operation of the vein side upon starting blood withdrawal (C)).

### (2) Automatic blood withdrawal process (blood withdrawal with high-performance water removing)

By blocking (clamping) the blood line near the artery and vein connecting parts 1, 2, e.g. with forceps, both connecting parts are detached and connected to the paracentesis needles (AFV etc.) pierced into the patient body after removing the air remained in the connecting parts. After removing the forceps blocking the line, the switch button for automatic blood withdrawal is pressed thereby shifting the operation mode into automatic blood withdrawal mode. Further, in order to prevent the mixing of air bubbles into the patient side, the operation mode may also be shifted into automatic blood withdrawal mode by, prior to starting blood withdrawal, closing the clamp PV2 below the venous chamber to start blood withdrawal in a water removing method, and by opening the foregoing clamp below the venous chamber after 1 to 3 seconds.

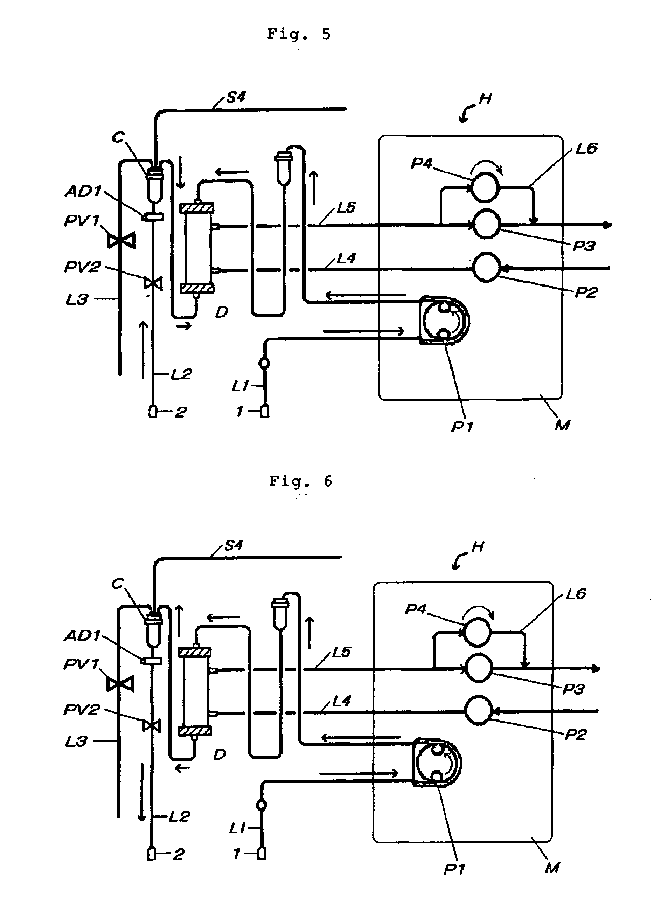

With the valve 1 (PV1) closed and the valve 2 (PV2) opened, the water removing/liquid replenishing pump P4 is rotated at 100 ml/min (internal setting) to remove water from the dialysate. Also, the blood pump P1 is rotated in the forward direction at 50 ml/min (internal setting). An amount of water to be removed or a time period until water has been removed from the filling liquid (dialysate) in the blood line through the hemodialyzer and the filling liquid (dialysate) in the blood line is replaced by blood is set (for example 1 min) in advance, and blood withdrawal is to be completed when the predetermined amount of water to be removed or the predetermined time period has been reached. As soon as the blood withdrawal is completed, the operation mode is shifted to a normal hemodialyzing mode automatically or manually (Fig. 5). In the case of the present blood withdrawal process, blood is withdrawn from the artery at a rate of 50 ml/min by the rotation of the blood pump P1 at 50 ml/min (internal setting), and also the amount of blood corresponding to the difference in the rotation between the water removing/liquid replenishing pump P4 and the blood pump P1, that is 50 ml/min (100 ml/min - 50 ml/min), is withdrawn from the vein.

### (3) Automatic blood withdrawal process (by overflow discharge)

As with the above described blood withdrawal with high-performance water removing, while the blood line is blocked (clamped) near the artery/vein connecting parts 1, 2, e.g. by means of forceps or the like, both connecting parts 1, 2 are detached and connected to the paracentesis needles (AVF etc.) pierced into the patient body after removing the air existing in the connecting parts. Then, operations of removing the forceps blocking the line, and pressing the automatic blood withdrawal button thereby shifting the operation mode into an automatic blood withdrawal mode are performed.

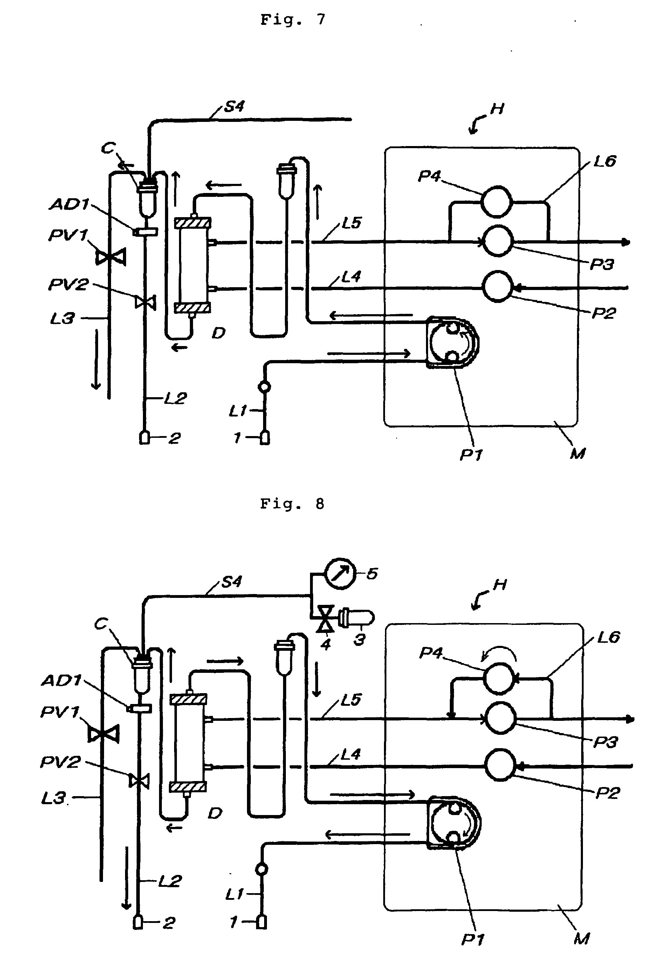

With the valve 1 (PV1) opened, the valve 2 (PV2) closed, and the water removing/liquid replenishing pump P4 being at a halt, the blood pump P1 is forwardly rotated at 100 ml/min (internal setting). Thereafter, as with the blood withdrawal process with high-performance water removing, blood withdrawal process is continued until the filling liquid (dialysate) in the blood line is replaced with blood (Fig. 7).

In the above described blood withdrawal process, the variation in the vein pressure was measured with the venous line L2 being blocked for 1 to 3 seconds by means of the clamp PV2 provided in the venous Line L2 in the downstream of the venous chamber C, with the blood pump being kept operating in the foregoing blocked state, and with the water removing pump being stopped. When the blood withdrawal is being performed normally, the vein pressure will rise as the blood pump operates, while in a state of blood withdrawal failure, the vein pressure will not rise even though the rotational speed of the blood pump increases. Utilizing such variation of the vein pressure, a blood withdrawal failure in the arterial side is detected and a warning is generated. Further, for detecting blood withdrawal failure in the venous side in the blood withdrawal process, the blood pump is temporally stopped and the water removing speed is reduced by a speed corresponding to the speed of the blood pump to continue blood withdrawal in the venous side. When the vein pressure is reduced lower than a lower limit or the liquid pressure becomes lower than a predetermined lower limit, it is determined that a blood withdrawal failure in the venous side has occurred, and the process is controlled either to automatically shift to blood withdrawal from the arterial side alone or to stop and generate a warning.

Further, when the dialysate pressure becomes an excessively negative pressure state (an excessively negative pressure state is measured by providing a pressure sensor in the dialysate discharge side, and the pressure reference corresponding to the excessively negative pressure can be altered by internal setting) after starting blood withdrawal operation, it is determined that a blood withdrawal failure has occurred and the process is controlled either to automatically shift to blood withdrawal with water removing from the arterial s ide alone or to stop and generate a warning.

### (4) Hemodialysis process

In the hemodialysis process, with valve 1 (PV1) closed and the valve 2 (PV2) opened, the blood pump P1 and the water removing/liquid replenishing pump P4 are forwardly driven at a predetermined flow rate. The valve 2 (PV2) operates in conjunction with the air-bubble detector AD1, and is closed as soon as an air-bubble is detected in the blood line. The blood pump P1 is also stopped (Fig. 6). Further, the valve 2 (PV2) operates also in conjunction with the air-bubble detector AD2, and is to be closed upon detecting an air-bubble in the blood line. The blood pump P1 is also stopped. Moreover, with a vein pressure monitor line, the above described hemodialysis process is configured such that when the measure of the vein pressure does not vary within a predetermined range in about 30 to 60 seconds after starting hemodialyzing, an alarm is generated indicating that an obstruction has occurred in the vein pressure monitor line S4.

### (5) Automatic blood returning process

After the completion of the water removing or the elapse of the target hemodialysis time, operation is shifted to the automatic blood returning process by pressing the automatic blood returning button, or it is automatically shifted to the automatic blood returning process after the completion of the water removing or the elapse of the target hemodialysis time.

With valve 1 (PV1) closed and the valve 2 (PV2) opened, the water removing/liquid replenishing pump P4 is rotated at 200 ml/min (internal setting) for back-filtering the dialysate and the blood pump P1 is backwardly rotated at 100 ml/min (internal setting) which is 50 % of the rate of the water removing pump. By the abovementioned ratio of flowrate, that is blood is returned from the arterial side at a rate of 100 ml/min by the pump P1, and from the venous side at a rate of 100 ml/min which is determined by the difference in the rotational speed between the water removing/liquid replenishing pump P4 and the foregoing blood pump P1 (200 ml/min-100 ml/min). After the predetermined amount of back-filtration or the predetermined time has reached, or when the remaining blood in the blood line has been replaced with dialysate (cleaning liquid), the blood pump P1 and the water removing/liquid replenishing pump P4 are stopped completing the automatic blood returning process (Fig. 8).

When, for some reasons, blood returning only from the vein side is des ired, it is poss ible to return much of the blood remained in the arterial line from the vein side by providing a bypass line for communicating from the artery side to the vein side and closing the arterial blood line between the connecting part of the bypass line and the artery side connecting part 1.

The above described automatic blood returning process was performed, as with the case of liquid pressure feedback control in the automatic priming process, by automatically setting an appropriate back-filtration speed through a feedback control of the back-filtration speed based on the measurement of dialysate pressure in the automatic blood returning process. Further, the automatic blood returning process was performed while monitoring the presence of abnormal internal pressure in the line during blood returning, by means of the function of detecting abnormal internal pressure in the line during blood returning which is capable of measuring the dialysate pressure in the dialysate line and the vein pressure in the venous line with pressure measuring means to determine that an obstruction of the line has occurred when the vein pressure in the venous line goes into an increasing trend from a stable state and also when both of the dialysate pressure and the vein pressure in the venous line go into an increasing trend from a stable state.

### INDUSTRIAL APPLICABILITY

(1) According to the hemodialyzing apparatus of the present invention, not only it is made possible to automate the priming process by cooperatively controlling the blood pump provided in the blood circulation system, the back-filtration speed regulation means, the line open/close means provided in the overflow line and the blood line in the downstream of the blood chamber, to facilitate the operation in the blood withdrawal process, to prevent the inflow of the priming liquid into the patient body, and further to safely, securely, and rapidly perform each process itself from hemodialysis preparation to completion of treatment, and most of transitions from a certain process to next process compared to the conventional hemodialyzing apparatuses, for example the invention described in JP, A, 2000-325470, but also it is made possible to automate by programming a series of treatment processes including blood withdrawal/hemodialysis starting/recovery/treatment completing processes.
(2) Due to the effects described in the above (1), it was made possible to significantly reduce labor and consumable costs. That is, the hemodialyzing apparatus according to the present invention will significantly reduce the amount of time spent by medical personnel at bedside, and contributes to the enhancement of efficiency and labor saving in medical practices relating to hemodialysis treatment.
(3) It is possible to further enhance the effects listed in (1) and (2), by providing the above described functions (A) to (K) in addition to means of cooperatively controlling the blood pump, the back-filtration speed regulation means, the line open/close means provided in the overflow line and the blood line on the downstream side of the blood chamber.
(4) The hemodialyzing apparatus of the present invention makes it possible to perform blood withdrawal from artery side alone, concurrent blood withdrawal from both artery and vein sides, and overflow line automatic blood withdrawal by controlling the blood pump, the back-filtration speed regulation means, and the open/close means provided in the overflow line and the line on the downstream side of the connection part of the vein chamber.
(5) It is possible effectively perform cleaning and air-bubble removal of the blood line in the priming process. It is also possible to rapidly replenish any amount of dialysate at any speed (by the above described back-filtration) by supplying the dialysate to the blood line by operating the back-filtration speed regulation means during hemodialysis. Further, after the completion of hemodialysis,it is possible to return the blood in the arterial blood line and venous blood line into the patient body through the above described back-filtration without detaching the blood line by cooperatively controlling the back-filtration speed regulation means and the blood pump. Furthermore, it is possible to prevent the mixing of air, inadvertent needling, blood pollution, and the like during the blood returning process, to reduce the burden of the medical staff, and to rapidly and safely perform blood returning.

## Claims

1. An automatic hemodialyzing apparatus composed of at least:
(a) a hemodialyzer for purifying blood by bringing blood and dialysate into contact via a semi-permeable membrane;
(b) a blood circulation system for circulating blood, the blood circulation system consisting of an arterial line for deriving blood from the patient to feed it into the hemodialyzer, and a venous line for returning blood, which has flown out from the hemodialyzer, to the patient;
(c) a dialysate supply/discharge system for supplying/discharging dialysate, the dialysate supply/discharge system having a dialysate supply line for supplying dialysate to the hemodialyzer, and a dialysate discharge line for discharging the dialysate which has flown out of the hemodialyzer, and
(d) back-filtration speed regulation means, wherein said automatic hemodialyzing apparatus comprises:
a blood pump in at least one of the two blood lines of said blood circulation system, the blood pump being reversibly-rotatable and adjustably cooperate with said back-filtration speed regulation means (d);
a venous chamber in the venous line;
an overflow line linked to said venous chamber and having open/close means for allowing liquid to overflow from said vein chamber to outside the blood line; and
line open/close means in a downstream side line and/or an upstream side line of the linking part of said venous chamber,
and also said automatic hemodialyzing apparatus has at least one function selected from the group consisting of following (A) to (K):
(1) function of detecting blood line obstruction during priming (A),
(2) function of back-filtration for canceling negative pressure in the line during priming (B),
(3) function of operating line open/close means in the downstream of the venous chamber upon starting blood withdrawal (C),
(4) function of controlling back-filtration of the dialysate in at least one of priming process, liquid replenishing process and blood returning process (D),
(5) function of blood-withdrawal failure detection (E),
(6) function of detecting obstruction of the vein pressure monitor line during hemodialysis (F),
(7) function of rapid liquid-replenishing in both the arterial and venous directions, or in the vein direction (G),
(8) function of detecting abnormal internal pressure in the line during blood returning (H),
(9) function of automatic regulation of the liquid level in the venous chamber (I),
(10) function of hemodialyzer exchange (J), and
(11) function of liquid discharge operation during blood line recovery/disposal (K).

2. The automatic hemodialyzing apparatus according to claim 1, **characterized by** at least having the function of controlling back-filtration of the dialysate in at least one of priming process, liquid replenishing process and blood returning process (D), the function of blood-withdrawal failure detection (E), and the function of detecting abnormal internal pressure in the line during blood returning (H).

3. The automatic hemodialyzing apparatus according to claim 1 or 2, **characterized in that** each process of the priming process before starting hemodialysis, blood withdrawal process from the patient to the blood circulation system upon starting hemodialysis, hemodialysis process, and blood returning process from the blood circulation system to the patient upon completing hemodialysis can be performed, or each of these processes can be performed automatically in a continuous manner, by controlling the blood pump, the back-filtration speed regulation means, the overflow line, and the line open/close means provided in the downstream side line and/or the upstream side line of the connecting part of the venous chamber.

4. The automatic hemodialyzing apparatus according to claim 1, 2, or 3, **characterized in that** an air-bubble sensor is provided in the blood line.

5. The automatic hemodialyzing apparatus according to claim 1, 2, 3, or 4, **characterized by** comprising a venous monitoring line.

6. The automatic hemodialyzing apparatus according to claim 1, **characterized in that** the function of detecting blood line obstruction during automatic priming (A) is a function of enabling the air-bubble sensor for a fixed period of time in the automatic priming process to check if the blood line is filled with the priming liquid and determining that a blood line obstruction has occurred upon detecting air.

7. The automatic hemodialyzing apparatus according to claim 1, **characterized in that** the function of back-filtration for canceling negative pressure in the line during automatic priming (B) is a function of periodically performing a small amount of back-filtration in a state that the priming liquid is circulating in blood line during automatic priming to prevent the tendency of negative pressure in the blood line.

8. The automatic hemodialyzing apparatus according to claim 1, **characterized in that** the function of operating line open/close means in the downstream of the venous chamber upon starting blood withdrawal (C) is a function of, after completing the priming process, starting the water removing/blood withdrawal (blood withdrawal process B) by closing the venous line with the line open/close means provided in the downstream of the venous chamber before starting automatic blood withdrawal, and opening said venous line in a predetermined time after the blood withdrawal to make the pressure in the blood line negative and thereby prevent the mixing of air-bubbles into the patient side.

9. The automatic hemodialyzing apparatus according to claim 1, **characterized in that** the function of controlling back-filtration of the dialysate in at least one of priming process, liquid replenishing process and blood returning process (D) is a feedback control (also referred to as constant pressure control system) of the back-filtration speed based on the liquid pressure of the hemodialyzer.

10. The automatic hemodialyzing apparatus according to claim 9, **characterized in that** the function of controlling back-filtration (D) is a feedback control in which a back-filtration speed, which is set after the automatic priming process has been stabilized, is stored as a maximum value and used as the maximum value for the back-filtration speed of liquid pressure feedback control in the automatic liquid replenishing and automatic blood returning processes.

11. The automatic hemodialyzing apparatus according to claim 1, **characterized in that**, the function of controlling back-filtration (D) may be such a control system as that at least in one of the automatic priming process, the liquid replenishing process and the blood returning process, a set value for liquid pressure control of said process is predefined and every time a liquid pressure measurement reaches the set value, the set back-filtration speed is decreased at a fixed percentage or by a fixed speed (also referred to as speed control system).

12. The automatic hemodialyzing apparatus according to claim 9, 10, or 11, **characterized in that** the function of controlling back-filtration (D) is optionally selectable between the constant pressure control system based on liquid pressure and the speed control system, or a combination thereof.

13. The automatic hemodialyzing apparatus according to claim 1, **characterized in that** the function of blood-withdrawal failure detection (E) is a function which, in the blood withdrawal or hemodialysis process, closes the circuit line for a fixed period of time with the line open/close means provided in the downstream of the venous chamber of the venous line to detect the variation of the vein pressure by operating the blood pump in said closed state, and determines the occurrence of an artery side blood-withdrawal failure based on the detection result.

14. The automatic hemodialyzing apparatus according to claim 1, **characterized in that** the function of blood-withdrawal failure detection (E) is a function of starting the blood withdrawal operation from the vein side to determine that the vein side is in a blood-withdrawal failure state when dialysate pressure becomes a negative pressure state below a fixed value (internal setting).

15. The automatic hemodialyzing apparatus according to claim 1, **characterized in that** the function of blood-withdrawal failure detection (E) includes a function of detecting a vein side blood-withdrawal failure, which temporally stops the blood pump and continues vein side blood withdrawal with the water removal speed being lowered by the speed of the blood pump and, when, in this state, the vein pressure is below a lower limit value or the liquid pressure is below a set lower limit value, determines the occurrence of a blood-withdrawal failure.

16. The automatic hemodialyzing apparatus according to claim 1, **characterized in that** the function of detecting obstruction of the vein pressure monitor line during hemodialysis (F) is a function of determining that an obstruction in the vein pressure monitor line has occurred when the vein pressure measurement does not vary within a predetermined range in a predetermined time period after starting hemodialysis.

17. The automatic hemodialyzing apparatus according to claim 1, **characterized in that** the function of detecting abnormal internal pressure in the line during blood returning (H) is a function of measuring dialysate pressure in the dialysate line and vein pressure in the venous line with pressure measuring means after starting blood withdrawal in the blood withdrawal process to determine that an abnormal internal pressure in the line has occurred when the vein pressure in the venous line shows a rising trend from a stable state.

18. The automatic hemodialyzing apparatus according to claim 1, **characterized in that** the function of automatic regulation of the liquid level in the venous chamber (I) allows a control with which the liquid level is automatically lowered during blood returning.

19. The automatic hemodialyzing apparatus according to claim 1, **characterized in that** the function of hemodialyzer exchange (J) is a function of reversely rotating the blood pump when a leakage in the hemodialyzer is found and keeping the blood to be pushed out in both artery and vein directions with a blood returning operation by back-filtration to exchange the hemodialyzer.

20. The automatic hemodialyzing apparatus according to claim 1, **characterized in that** the function of liquid discharge operation during blood line recovery/disposal (K) is a function in which, after completing blood returning, both tips of artery and venous lines are bypassed each other and the overflow line is detached or the open/close means provided in the overflow line is opened to draw out the saline in the line by performing water removing operation, and thereafter dialysate in the hemodialyzer is drawn out to the dialysate supply/discharge system (console) side utilizing the fall thereby reducing the weight of blood line wastes.

21. A priming method of a hemodialyzing apparatus, **characterized in that** said priming method comprises:
linking the arterial line and the venous line of the hemodialyzing apparatus according to claim 1, 2, 3, 4, 5, 6, 7, 8, 9, 10, 11, 12, 13, 14, 15, 16, 17, 18, 19, or 20; and
performing the priming operation by controlling the blood pump, the third liquid feed means, the line open/close means provided in the overflow line, and the line open/close means provided in the upstream line or the downstream line of the overflow line linking part.

22. The priming method of a hemodialysing apparatus according to claim 21, **characterized in that** the priming operation is performed by using dialysate.

23. A method of reducing the weight of blood line after hemodialysis, **characterized in that** the method comprising:
completing a series of hemodialysis operations from the priming operation to the blood returning process for returning the blood from the blood circulating system to the patient by using the hemodialyzing apparatus according to claim 1, 2, 3, 4, 5, 6, 7, 8, 9, 10, 11, 12, 13, 14, 15, 16, 17, 18, 19, or 20; thereafter,
drawing out saline in the line by bypassing each other the tips of both artery and venous lines and detaching the overflow line or opening the open/close means provided in the overflow line and performing water removing operation; and thereafter,
drawing out dialysate in the hemodialyzer to the dialysate supply/discharge system (console) utilizing the fall.
